# EUROPEAN PATENT SPECIFICATION

(11) **EP 1 639 002 B1**
(45) Date of publication and mention of the grant of the patent: **05.05.2010**
(21) Application number: 04743175.4
(22) Date of filing: 28.06.2004
(51) Int. Cl.: C07K 14/52, C07K 14/61, C12N 15/10, C12N 15/86, A01K 67/027, A61K 38/27, A61K 38/19, A61K 48/00

(54) **CYTOKINE VARIANT POLYPEPTIDES**
CYTOKINPOLYPEPTIDVARIANTEN
POLYPEPTIDES VARIANTS DE CYTOKINE

(30) Priority: 28.06.2003 GB 0315182
(43) Date of publication of application: 29.03.2006
(73) Proprietor: Asterion Limited, Sheffield S10 2TN (GB)
(72) Inventor: SAYERS, Jon, Asterion Limited, Sheffield S10 2TN (GB); ARTYMUIK, Peter, Asterion Limited, Sheffield S10 2TN (GB); ROSS, Richard, Asterion Limited, Firth Court, Sheffield S10 2TN (GB)
(74) Representative: Harrison IP Limited
(86) International application number: PCT/GB2004/002827
(87) International publication number: WO 2005/003165

(56) References cited:
- WO-A-00/18905
- US-A- 5 635 599
- US-A- 6 136 563
- KREITMAN ROBERT J ET AL: "Circularly permuted interleukin 4 retains proliferative and binding activity" CYTOKINE, vol. 7, no. 4, 1995, pages 311-318, XP002326238 ISSN: 1043-4666 cited in the application
- HORLICK R A ET AL: "PERMUTEINS OF INTERLEUKIN 1 BETA-A SIMPLIFIED APPROACH FOR THE CONSTRUCTION OF PERMUTATED PROTEINS HAVING NEW TERMINI" PROTEIN ENGINEERING, OXFORD UNIVERSITY PRESS, SURREY, GB, vol. 5, no. 5, 1992, pages 427-431, XP002022097 ISSN: 0269-2139
- MCWHERTER C A ET AL: "Circular permutation of the granulocyte colony-stimulating factor receptor agonist domain of myelopoietin" BIOCHEMISTRY, AMERICAN CHEMICAL SOCIETY. EASTON, PA, US, vol. 38, no. 14, 6 April 1999 (1999-04-06), pages 4564-4571, XP002131431 ISSN: 0006-2960

## Description

The invention relates to polypeptides comprising human growth hormone wherein at least one binding site for human growth hormone receptor is disrupted; oligomers thereof and their use as pharmaceutical agents.

A large group of growth factors, referred to as cytokines, are involved in a number of diverse cellular functions. These include, by example and not by way of limitation, modulation of the immune system, regulation of energy metabolism and control of growth and development. Cytokines mediate their effects via receptors expressed at the cell surface on target cells. Cytokine receptors can be divided into four separate sub groups. Type 1 (growth hormone (GH) family) receptors are characterised by four conserved cysteine residues in the amino terminal part of their extracellular domain and the presence of a conserved Trp-Ser-Xaa-Trp-Ser motif in the C-terminal part. The repeated Cys motif are also present in Type 2 (interferon family) and Type III (tumour necrosis factor family).

It is known that many cytokine ligands interact with their cognate receptor via specific sites. Some cytokine receptors have both high affinity ligands binding sites and low affinity binding sites.

For example, it is known that a single molecule of GH associates with two receptor molecules (GHR) (Cunningham *et al*., 1991; de Vos *et al*., 1992; Sundstrom *et al*., 1996; Clackson *et al*., 1998). This occurs through two unique receptor-binding sites on GH and a common binding pocket on the extracellular domains of two receptor. Site 1 on the GH molecule has, a higher affinity than site 2, and receptor dimerization is thought to occur sequentially with one receptor binding to site 1 on GH followed by recruitment of a second receptor to site 2. The extracellular domain of the GHR exits as two linked domains each of approximately 100 amino acids. It is a conformational change in these two domains that occurs on hormone binding with the formation of the trimeric complex GHR-GH-GHR. Internalisation of the GHR-GH-GHR complex is followed by a recycling step whereby the receptor molecule is regenerated for further use within the cell.

Variant growth hormone polypeptides are known. For example, GH variants are disclosed in US 5, 849, 535. The modification to GH is at both site 1 and site 2 binding sites. The modifications to site 1 produce a GH molecule that has a higher affinity for GHR compared to wild-type GH. These modified GH molecules act as agonists. There is also disclosure of site 2 modifications that result in the creation of GH antagonists. Further examples of modifications to GH which alter the binding affinity of GH for site 1 are disclosed in US 5,854,026; US 6,004,931; US6,022,711; US6,057,292; and US6136563. These modifications relate to point mutations at specific positions in GH which produce a molecule with altered signalling properties.

Circular permutation is a means to generate polypeptide variants that retain the overall tertiary structure of a native polypeptide but re-orders the primary linear sequence by forming new amino and carboxyl termini. The process generates molecules with altered biological properties. The process includes the fusion of the natural amino and carboxyl termini either directly or by using linker molecules that are typically peptide linkers. The conceptually circularised molecule is then cut to generate new amino and carboxyl termini. Circularly permuted polypeptides can be generated either recombinantly or by *in vitro* peptide synthesis.

Circular permutation has been used to generate chimeric molecules with altered biological activity.

For instance, WO95/27732 discloses the creation of a circularly permuted IL-4 ligand fused to a cytotoxic agent. The permuted IL-4-agent has altered affinity and cytotoxicity when compared to a native IL-4-agent and has efficacy with respect to killing cancer cells which are exposed to the conjugated polypeptide.

WO99/51632 describes the use of circular permutation to generate novel streptavidin binding proteins that have reduced affinity for biotin. The circularly permuted streptavidin is fused to a second polypeptide to create a fusion protein that differential ly binds biotin. The reduced affinity of the strepavidin fusion protein for biotin facilitates release of the fusion protein when biotin is used as a drug delivery vehicle.

WO01/51629 discloses circularly permuted bacterial β-lactamase and its use as a marker protein for the detection of interactions between intracellular and extracellular proteins that assemble with the permuted polypeptide.

Methods to identify circularly permuted polypeptides are also known. For example; WO00/18905, which is incorporated by reference, describes a method to identify permuted polypeptides, referred to as "permuteins", using a phage display vector into which a library of permuted genes are inserted. The expression of the library at the surface of the display vector is detected by exposure of the expressed library to a binding protein that potentially interacts with a permutein.

WO01/30998, which is incorporated by reference, discloses a further method to generate and identify circularly permuted proteins. The invention relates to the formation of fusion proteins comprising the amino terminal part of a first protein fused to the carboxyl terminal part of a different second protein from which permuteins are synthesised. A library of fusion proteins is created which can be screened by phage display.

We have applied the process of circular permutation to human growth hormone to generate human growth hormone variants that are "gapped" at binding sites for their receptors to produce human growth hormones with altered properties.

We have generated a series of circularly permuted GH constructs in which the formation of new amino and carboxyl terminal termini is localised to site 2 of GH thereby disrupting the low affinity binding site for ligand binding to the GHR.

We also disclose oligomers (tandems, trimers, etc) of said permuted polypeptides that have additional properties, for example delayed clearance from the circulatory system. The *in vivo* efficacy of many cytokines, for example GH, is determined in part by the affinity for GHR and rate of clearance from the circulation. Permuted polypeptides may also be fused to the extracellular binding domain of their cognate receptor(s). For example, we describe in PCT/GB01/02645; WO01/096565, the fusion of growth hormone to growth hormone receptors (for example the fusion of GH to GHR via linker molecules) that retain biological activity and have the advantageous property of delayed clearance.

The kidneys are relatively small organs that receive approximately 25% of cardiac output. The kidneys perform several important functions primarily related to the regulation of the composition and volume of body fluids. The kidneys filter about 100 litres of plasma every day and of the blood flow in and out of a kidney only approximately 1% becomes urine. Approximately 20% of the plasma that passes through the kidney gets filtered into the nephron. Filtration takes place in the glomerulus that is driven by the hydrostatic pressure of the blood. Water and small molecules are filtered whereas blood cells and large molecules, for example polypeptides, do not pass through the glomerular filter. Those polypeptides with an effective molecular weight above 70 kDa are not cleared by glomerular filtration because they are simply too large to be filtered. Certain proteins of small molecular weight are filtered by the glomerulus and are found in the urine. For example, GH has a molecular weight of 22.1 kDa and the kidney is responsible for clearing up to 60-70% of GH in humans (Baumann, 1991; Haffner et al, 1994), and up to 67% in rat (Johnson & Maack, 1977).

A modified human growth hormone polypeptide is provided comprising a modified amino acid sequence which is a modification of the native human growth hormone amino acid sequence, wherein the native amino terminal and carboxyl terminal amino acid residues of native human growth hormone are linked, directly or indirectly, together, wherein the modified human growth hormone is provided with alternative amino terminal and carboxyl terminal amino acid residues and further wherein at least one binding domain of human growth hormone is disrupted.

In a further preferred embodiment of the invention said native amino terminal and carboxyl terminal amino acid residues are directly linked to each other.

In an alternative preferred embodiment of the invention said native amino terminal and carboxyl terminal amino acid residues are indirectly linked by a linking molecule. Preferably said linking molecule is a peptide linkage.

In a preferred embodiment of the invention said linking peptide is a flexible peptide linker.

Preferably the flexible linker is a polypeptide that comprises 5 to 30 amino acid residues. More preferably the linker comprises 10 to 20 amino acid residues. More preferably still the linker comprises at least one copy of the peptide:
Gly Gly Gly Gly Ser (hereinafter referred to as "Gly4Ser").

In one embodiment of the invention the linker is 10 amino acids in length and comprises two copies of the Gly4Ser linker. In an alternative embodiment of the invention, the linker is 15 amino acids in length and comprises three copies of the Gly4Ser linker. In yet an alternative embodiment, the linker is 20 amino acids in length and comprises four copies of the Gly4Ser linker.

In an alternative embodiment of the invention said linker is an inflexible linker, for example a linker wherein said linker is, over part of its length, has a α-helical region.

For example, Arai et al (Protein Eng 14(8): 529-532 (2001) investigated the use of helix-forming peptides A(EAAAK)ₙA to separate domains of different green fluorescent proteins and measured the separation of the proteins using FRET, showing such linkers to behave as rigid entities of fixed length. JP2002247997 utilises an α-helical linker sequence to link E6 ligand to IL-6 receptor. It is envisaged that linkers that have properties between the extremes of a flexible linker and a helical linker may also be utilised to link respective native ends of human growth hormone.

In a preferred embodiment of the invention said receptor binding domain low affinity binding domain site 2 of growth hormone.

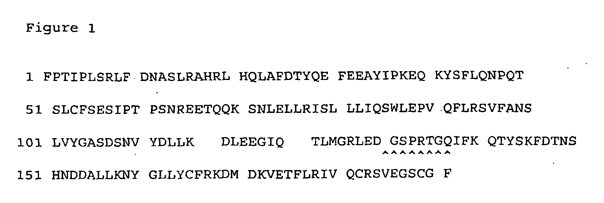

In a further preferred embodiment of the invention said low affinity binding domain of growth hormone is between about amino acid 116 - amino acid 122 of human growth hormone as represented by the amino acid sequence shown in Figure 1.

In a further preferred embodiment of the invention said alternative amino terminal and carboxyl terminal amino acid residues are derived from between amino acid 116 and amino acid 122 of human growth hormone as represented by Figure 1.

In a further preferred embodiment of the invention said alternative amino terminal and carboxyl terminal amino acid residues are derived from between amino acid 118 and amino acid 121 of human growth hormone as represented by Figure 1.

In a further preferred embodiment of the invention said alternative amino terminal and carboxyl terminal amino acid residues are derived from between amino acid 119 and amino acid 121 of human growth hormone as represented by Figure 1.

In a further preferred embodiment of the invention said alternative amino terminal and carboxyl terminal amino acid residues are derived from between amino acid 120 and ammo acid 121 of human growth hormone as represented by Figure 1.

In a further preferred embodiment of the invention said alternative amino terminal and carboxyl terminal amino acid residues are derived from between amino acid 118 and amino acid 120 of human growth hormone as represented by Figure 1.

In a further preferred embodiment of the invention said alternative amino terminal and carboxyl terminal amino acid residues are derived from between amino acid 119 and amino acid 120 of human growth hormone as represented by Figure 1.

In an alternative preferred embodiment of the invention said alternative amino terminal and carboxyl terminal amino acid residues are derived from between about amino acid 100 and amino acid 102 of human growth hormone as represented by the amino acid sequence shown in Figure 1

In a further alternative preferred embodiment of the invention alternative amino terminal and carboxyl terminal amino acid residues are derived from between about amino acid 130 - amino acid 132 of human growth hormone as represented by the amino acid sequence shown in Figure 1

In a further preferred embodiment of the invention there is provided an oligomeric cytokine ligand polypeptide comprising at least two modified human growth hormone polypeptides according to the invention wherein said human growth hormones are linked, either directly or indirectly, together.

In a preferred embodiment of the invention said ligands are linked by a flexible peptide linker molecule. In an alternative preferred embodiment of the invention said ligands are linked by an inflexible peptide linker molecule, preferably said linker molecule comprises a α-helical region.

In a preferred embodiment of the invention said oligomer comprises two modified human growth hormone polypeptides.

In a further preferred embodiment of the invention said said oligomer comprises, at least 3; 4; 5; 6; 7; 8; 9; or at least 10 modified human growth hormone polypeptides.

In a further preferred embodiment of the invention said oligomeric cytokine polypeptide comprises at least two modified growth hormone polypeptides as hereindescribed. Preferably said oligomeric growth hormone polypeptide comprises multiple human growth hormone polypeptides.

In an alternative preferred embodiment of the invention there is provided an oligomeric human growth hormone polypeptide comprising at least one modified human growth hormone polypeptide according to the invention linked, either directly or indirectly, to at least one native human growth hormone polypeptide from which said modified human growth hormone polypeptide was derived.

In a further alternative embodiment of the invention there is provided a modified human growth hormone polypeptide according to the invention linked to at least one extracellular human growth hormone binding domain of human growth hormone receptor.

In our co-pending application, WO01/096565, we disclose fusion proteins which translationally fuse the ligand binding domain of a cytokine to the extracellular receptor binding domain of said ligand via flexible peptide linkers. These fusion proteins have delayed clearance and agonist activity. The fusion of cytokine to cognate receptor provides an immunologically silent polypeptide which has a molecular weight which slows renal clearance. Modified human growth hormone polypeptides as hereindisclosed could also benefit from delayed clearance.

Peptide linkers that link cytokine ligand polypeptides to one another to form oligomeric polypeptides (dimers, trimers etc) and to cognate extracellular receptor binding domains are either flexible (e.g. Gly4Ser) or inflexible (e.g. α-helical) or intermediate (e.g. a combinational linker which is part helical) as described above. Linkers may also contain cleavage sites to provide oligomeric polypeptides with delayed release characteristics.

In a preferred embodiment of the invention said linker comprises a cleavage site, preferably a proteolytic cleavage site.

Preferably said cleavage site is sensitive to a serum protease or a matrix metalloprotease.

In a preferred embodiment of the invention said cleavage site comprises the amino acid sequence: LVPRGS, or variant thereof.

Preferably, said cleavage site comprises the amino acid sequence PGI(S), or variant thereof.

More preferably still said cleavage site comprises the amino acid sequence: LVPRGS PGI, or variant thereof.

Alternatively, said cleavage site comprises at least two copies of the amino acid sequence GGGGS, or functional variant thereof, which flank said cleavage site.

In a further preferred embodiment of the invention said cleavage site is sensitive to the serum protease thrombin.

According to a further aspect of the invention there is provided a nucleic acid molecule which encodes a modified human growth hormone polypeptide or an oligomeric modified human growth hormone polypeptide according to the invention.

According to a further aspect of the invention there is provided a vector comprising a nucleic acid molecule according to the invention.

In a preferred embodiment of the invention said vector is an expression vector adapted for eukaryotic gene expression.

Typically said adaptation includes, the provision of transcription control sequences (promoter/enhancer sequences) which mediate cell/tissue specific expression. These promoter sequences may be cell/tissue specific, inducible or constitutive.

Promoter is an art recognised term and, for the sake of clarity, includes the following features which are provided by example only, and not by way of limitation. Enhancer elements are cis acting nucleic acid sequences often found 5' to the transcription initiation site of a gene (enhancers can also be found 3' to a gene sequence or even located in intronic sequences and are therefore position independent). Enhancers function to increase the rate of transcription of the gene to which the enhancer is linked. Enhancer activity is responsive to *trans* acting transcription factors (polypeptides) which have been shown to bind specifically to enhancer elements. The binding/activity of transcription factors (please see Eukaryotic Transcription Factors, by David S Latchman, Academic Press Ltd, San Diego) is responsive to a number of environmental cues that include, by example and not by way of limitation, intermediary metabolites (e.g. glucose, lipids), environmental effectors (e.g. heat).

Promoter elements also include so called TATA box and RNA polymerase initiation selection (RIS) sequences which function to select a site of transcription initiation. These sequences also bind polypeptides that function, *inter alia,* to facilitate transcription initiation selection by RNA polymerase.

Adaptations also include the provision of selectable markers and autonomous replication sequences which both facilitate the maintenance of said vector in either the eukaryotic cell. Vectors which are maintained autonomously are referred to as episomal vectors.

Adaptations which facilitate the expression of vector encoded genes include the provision of transcription termination/polyadenylation sequences. This also includes the provision of internal ribosome entry sites (IRES) that function to maximise expression of vector encoded genes arranged in bicistronic or multi-cistronic expression cassettes.

These adaptations are well known in the art. There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour. NY and references therein; Marston, F (1987) DNA Cloning Techniques: A Practical Approach Vol will XRL Press, Oxford UK; DNA Cloning: F M Ausubel et al, Current Protocols in Molecular Biology, John Wiley & Sons, Inc. (1994).

It will be apparent to one skilled in the art that the vectors according to the invention could be gene therapy vectors. Gene therapy vectors are typically virus based. A number of viruses are commonly used as vectors for the delivery of exogenous genes. Commonly employed vectors include recombinantly modified enveloped or non-enveloped DNA and DNA viruses, preferably selected from baculoviridiae, parvoviridiae, picornoviridiae, herpesveridiae, poxviridae, adenoviridiae, or piconnaviridiae. Chimeric vectors may also be employed which exploit advantageous elements of each of the parent vector properties (See e.g., Feng, et al.(1997) Nature Biotechnology 1.5:866-87Q). Such viral vectors may be wild-type or may be modified by recombinant DNA techniques to be replication deficient, conditionally replicating or replication competent.

Preferred vectors are derived from the adenoviral, adeno-associated viral and retroviral genomes. In the most preferred practice of the invention, the vectors are derived from the human adenovirus genome. Particularly preferred vectors are derived from the human adenovirus serotypes 2 or 5. The replicative capacity of such vectors may be attenuated (to the point of being considered "replication deficient") by modifications or deletions in the E1a and/or Elb coding regions. Other modifications to the viral genome to achieve particular expression characteristics or permit repeat administration or lower immune response are preferred.

Alternatively, the viral vectors may be conditionally replicating or replication competent. Conditionally replicating viral vectors are used to achieve selective expression in particular cell types while avoiding untoward broad spectrum infection. Examples of conditionally replicating vectors are described in Pennisi, E. (1996) Science 274:342-343; Russell, and S.J. (1994) Eur. J. of Cancer 30A(8): 1165-1171.

Additional examples of selectively replicating vectors include those vectors wherein a gene essential for replication of the virus is under control of a promoter which is active only in a particular cell type or cell state such that in the absence of expression of such gene, the virus will not replicate. Examples of such vectors are described in Henderson, et al., United States Patent No. 5,698,443 issued December 16, 1997 and Henderson, et al., United States Patent No. 5,871,726 issued February 16, 1999. Vectors may also be non-viral and are available from a number of commercial sources readily available to a person -skilled in the art. For example, the vectors may be plasmids that can be episomal or integrating.

According to a yet further aspect of the invention there is provided a cell transfected or transformed with a nucleic acid molecule or vector according to the invention.

In a preferred embodiment of the invention said cell is a eukaryotic cell. Preferably said cell is selected from the group consisting of: a mammalian cell (e.g. Chinese Hamster Ovary cell); yeast cells (e.g. *Saccharomyces spp, Pichia* spp); insect cells (e.g. *Spodoptera spp*) or plant cells.

According to a yet further aspect of the invention there is provided a non-human transgenic mammal transfected/transformed with the nucleic acid molecule or vector according to the invention.

According to a yet further aspect of the invention there is provide a modified human growth hormone polypeptide, an oligomeric modified human growth hormone polypeptide, a nucleic acid molecule, a vector or a cell according to the invention for use as a pharmaceutical.

Preferably said ligand is an antagonist. In an alternative embodiment of the invention said ligand is an agonist.

Preferably said polypeptide, nucleic acid molecule, vector or cell is used in a pharmaceutical composition.

When administered the pharmaceutical composition of the present invention is administered in pharmaceutically acceptable preparations. Such preparations may routinely contain pharmaceutically acceptable concentrations of salt, buffering agents, preservatives, compatible carriers, and optionally other therapeutic agents.

The pharmaceutical composition of the invention can be administered by any conventional route, including injection. The administration and application may, for example, be oral, intravenous, intraperitoneal, intramuscular, intracavity, intra-articuar, subcutaneous, topical (eyes), dermal (e.g a cream lipid soluble insert into skin or mucus membrane), transdermal, or intranasal.

Pharmaceutical composition of the invention is administered in effective amounts. An "effective amount" is that amount of a pharmaceutical/composition that alone, or together with further doses or synergistic drugs, produces the desired response. This may involve only slowing the progression of the disease temporarily, although more preferably, it involves halting the progression of the disease permanently. This can be monitored by routine methods or can be monitored according to diagnostic methods.

The doses of the pharmaceutical composition administered to a subject can be chosen in accordance with different parameters, in particular in accordance with the mode of administration used and the state of the subject (i.e. age, sex). When administered, the pharmaceutical composition of the invention is applied in pharmaceutically-acceptable amounts and in pharmaceutically-acceptable compositions. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable, salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceuticauy-acceptable salts can be prepared as alkaline metal or alkaline earth salts, such as sodium, potassium or calcium salts.

The pharmaceutical composition may be combined, if desired, with a pharmaceutically-acceptable carrier. The term "pharmaccutically-acceptable carrier" as used herein means one or more compatible solid or liquid fillers, diluents or encapsulating substances that are suitable for administration into a human; The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction that would substantially impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride,; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier that constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous, liquids such as a syrup, elixir or an emulsion.

Compositions suitable for parenterat administration conveniently comprise a sterile aqueous or non-aqueous preparation that is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

Polypeptides/nucleic acid molecules etc according to the invention can be incorporated into liposomes. Liposomes are lipid based vesicles which encapsulate a selected therapeutic agent which is then introduced into a patient. The liposome is manufactured either from pure phospholipid or a mixture of phospholipid and phosphoglyceride. Typically liposomes can be manufactured with diameters of less than 200nm, this enables them to be intravenously injected and able to pass through the pulmonary capillary bed. Furthermore the biochemical nature of liposomes confers permeability across blood vessel membranes to gain access to selected tissues. Liposomes do have a relatively short half-life. So called STEALTH^{R} liposomes have been developed which comprise Liposomes coated in polyethylene glycol (PEG). The PEG treated liposomes have a significantly increased half-life when administered intravenously to a patient. In addition, STEALTH^{R} liposomes show reduced uptake in the reticuloendothelial system and enhanced accumulation selected tissues. In addition, so called immuno-liposomes have been develop which combine lipid based vesicles with an antibody or antibodies, to increase the specificity of the delivery of the agent to a selected cell/tissue.

The use of liposomes as delivery means is described in US 5580575 and US 5542935.

According to a further aspect of the invention there is provided a screening method to generate modified human growth hormone polypeptides according to the invention comprising the steps of:
i) forming a preparation comprising native cytokine ligand polypeptide molecules wherein the native amino terminal and carboxyl terminal amino acids are linked either directly or indirectly together;
ii) generating modified cytokine ligand polypeptide molecules wherein said molecules have alternative amino terminal and carboxyl terminal amino acids; and
iii) testing the activity of said modified cytokine ligand polypeptides.

Bioassays to test the activity of, for example growth hormone are known in the art and are disclosed in WO01/096565.

An embodiment of the invention will now be described by example only and with reference to the following figures:
Figure 1 shows the amino acid sequence of human GH with binding site for GHR indicated with arrows;

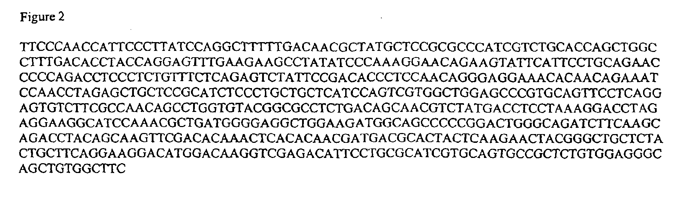

Figure 2 shows the nucleic acid sequence of human GH;

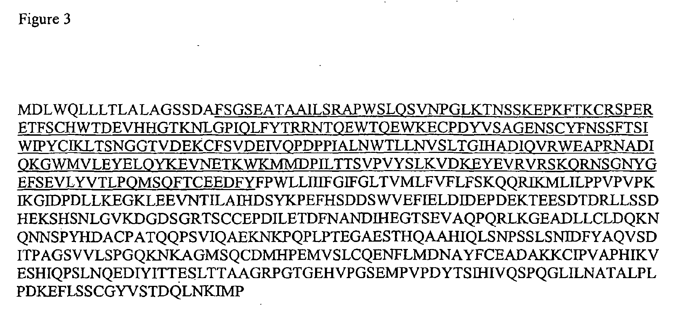

Figure 3 shows the amino acid sequence of GHR (extracellular domain underlined);
Figure 4 Cloning strategy for the circular permutation of Growth Hormone. In the first PCR reaction a forward primer (GH_CPFor) and a linker primer (GH_CPLink) are used to produce a 'megaprimer'. This megaprimer is used with a reverse primer (GH_CPRev) to generate the circularly permutated GH gene. Appropriate restriction sites [*Bam*HI (B) and *Not*I (N)] are engineered into the forward and reverse primers to facilitate ligation into the vector pTrcHis-TOPO;

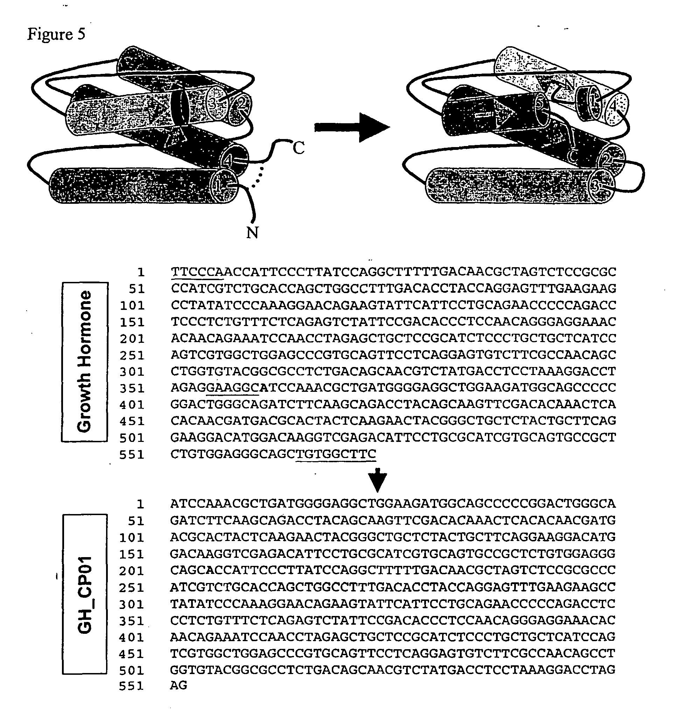

Figure 5: Schematic and DNA sequence showing the strategy used to generate GH_CP01. (A) a schematic diagram showing how GH is transformed into GH_CP01; Glu120 (grey disc) is removed by initiating the new gene at residue 121 and terminating the protein at residue 118, the 'old' termini are linked by joining the termini to make a 6 amino acid linker. The helices are numbered in order (from N to C terminus) and the arrows denote the direction of the helices (from N to C). (B) the DNA sequence of GH and GH_CP01; the nucleotides removed from GH to produce GH_CP01 are underlined, the initiation nucleotide for GH_CP01 in GH, and *vice versa,* are shown in bold;
Figure 6: Western blots showing the expression of GH_CP01 in three different systems. The blots show that GH_CP01 is expressed and detected by the antibody probes used in the western blot system utilised. T0 shows expression at the time of induction, T4 shows expression 4 hours post-induction, WP shows whole protein in the RTS reaction and SP shows only soluble protein in the RTS reaction. Protein produced from linear template lacks N-terminal His tag and adapter sequence and hence migrates slightly faster;

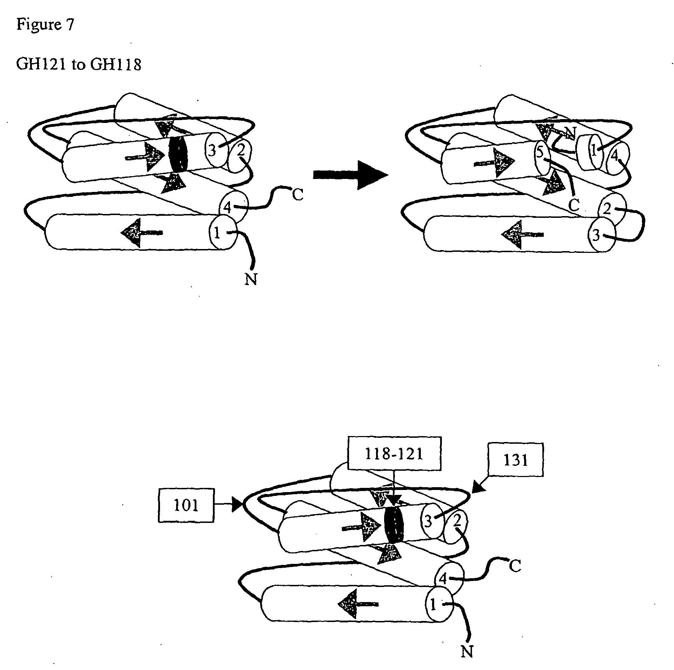

Figure 7 is a diagrammatic representation of circularly permuted GH molecules;

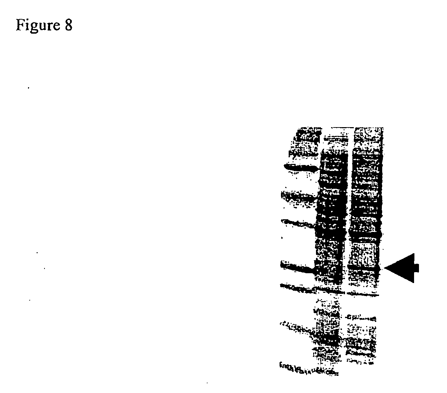

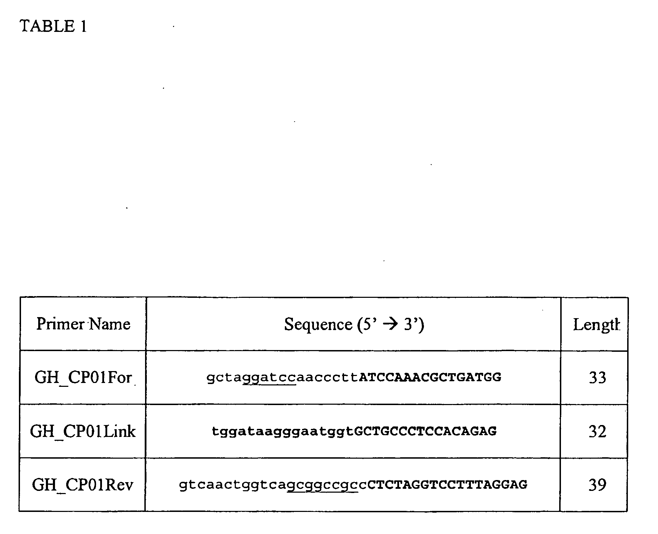

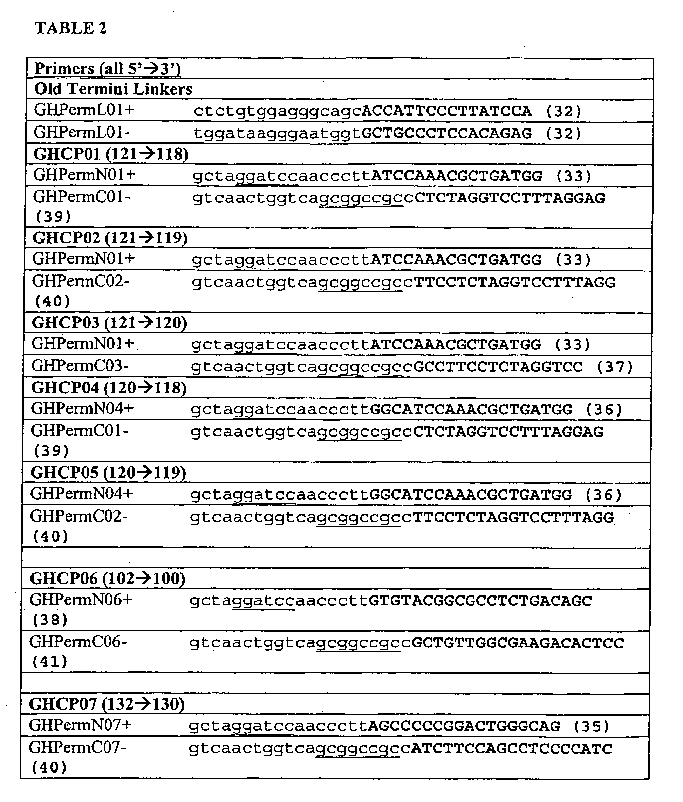

Figure 8 illustrates expression of CP02 protein in *E. coli.* The induced band of CP02 protein is denoted by the arrowhead. Lane 1: BioRad protein marker; Lane 2: Non-induced cells carrying GHCP02 construct in LB media; Lane 3: Induced cells carrying GHCP02 construct;
Table 1 Primers used to generate GH_CP01. The bold characters denote sequence that anneals to the GH gene; the underlined characters denote endonuclease restriction sites (*Bam*HI - ggatcc; *Not*I - gcggccgc). In the linker primer, GH_CP01Link, the sequence that anneals to the carboxyl terminus of GH is shown in UPPER-CASE; and
Table 2 Primers used to generate further circularly permuted GH molecules.

### Materials and Methods

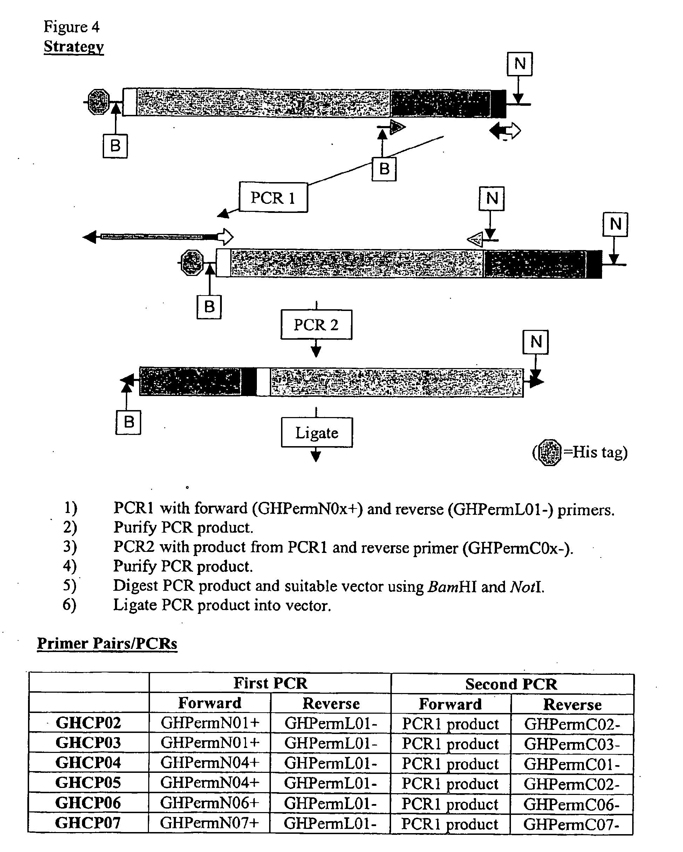

Growth hormone was circularly permutated by using a 2-step PCR methodology. The first PCR reaction generated a DNA fragment encoding the new amino terminus to the end of the GH gene and an overhang which could anneal to the start of the GH gene. This PCR product was then used as a 'megaprimer' in another PCR reaction to generate the full length GH_CP gene. The relevant primers were designed with restriction digestion sites for ligation into an appropriate vector (Figure 4).

The GH_CP gene was ligated into an expression vector and this transformed into an appropriate strain of *E. coli.* Expression of GH_CP was confirmed by western blot of protein from induced cultures of *E. coli* containing the GH_CP expression plasmid, the blots were probed using mouse anti-GH antibodies (10A7, mouse IgG1) and Sheep anti-mouse-HRP antibodies (Amersham).

The GH_CP protein was purified from cell lysates using a metal chelate affinity column (Probond resin, Invitrogen) followed by an ion exchange column (MonoQ, Pharmacia).

### Generation of the GH CP01 gene

The first embodiment of the growth hormone circular permutation is GH_CP01. The amino terminus of this construct initiated at residue Ile121 of GH and the carboxy terminus was at residue Glu118 of GH. The 'old' termini of GH were linked by a 6 amino acid linker, which was formed by joining the 'old' termini -3 amino acids from the first helix at the amino terminus and +3 residues from the last helix at the carboxy terminus (Figure 5)

Two PCR reactions were required to generate the circularly permutated GH gene. Three primers were designed (Table 1), GH_CP01For consisted of a *Bam*HI restriction endonuclease site followed by the DNA sequence of the new amino terminus of the permutated GH; GH_CP01Rev was an antisense primer consisting of the DNA sequence of the new carboxy terminus of the permutated GH and a *Not*I restriction endonuclease site; GH_CP01Link was an antisense primer designed to anneal to both termini of GH.

The primers GH_CP01For and GHCP01Link and the template pTrcHisGHstop were used in the first PCR reaction to produce a 'megaprimer'. This megaprimer was purified from the PCR reaction and then used in conjunction with GHCP01Rev, again using pTrcHisGHstop as the template, to produce the full length gene for the circularly permutated GH (GH_CP01). This gene was then ligated into the pTrcHis-TOPO plasmid vector between the *Bam*HI and *Not*I restriction endonuclease sites. The vector was then transformed into *E. coli* XL1 Blue cells. The success of the circular permutation of GH was confirmed by DNA sequencing of the pTrcHis.GH_CP01 plasmid.

### Expression of the GH CP

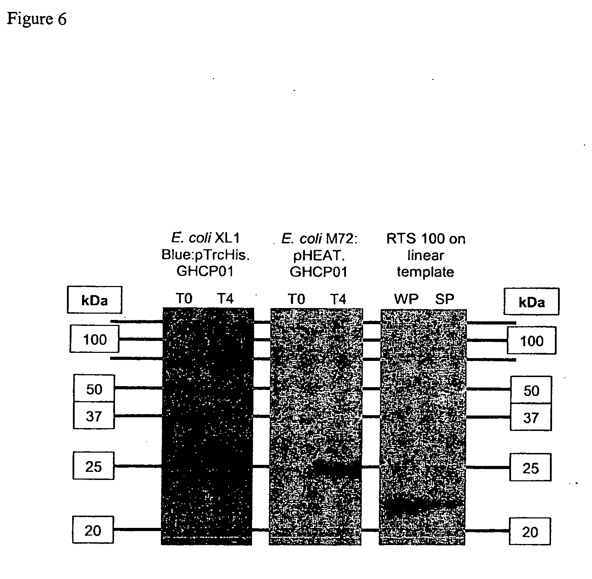

A variety of expression systems were used to optimise the production of GH CP01. Expression was carried out from the *E. coli* XL1 Blue:pTrcHis.GH_CP01 cells induced with IPTG. The GH_CP01 gene was also subcloned into the pHEAT vector and the resulting *E. coli* M72:pHEAT.GH_CP01 cells induced by thermal regulation. Cell free in vitro translation was also used for protein expression; both linear template and the gene subcloned into the pIVEX-23MCS vector were used to express GH_CP01 in the RTS system (Roche). All these systems produced soluble protein (Figure 6).

### Purification of GH CP

GH_CP01 protein was purified from *E. coli* XL1 Blue:pTrcHis.GH_CP01. The cells were harvested from the induced growth cultures were resuspended in Resuspension Buffer (20mM sodium phosphate buffer, 500mM sodium chloride, 5% glycerol, 25µg/ml PMSF, pH 7.8) were lysed by a 30 minute incubation on ice after the addition of 100µg/ml (final concentration) hen egg white lysozyme, 250µg/ml (final concentration) of sodium deoxycholate was then added and the solution incubated for a further 30 minutes on ice, the lysed cells were then sonicated. Insoluble material was removed by centrifugation at 19000rpm for 30 minutes.

The cleared cell lysate was applied to a 5ml Probond resin column (Invitrogen) which had been charged with Co²⁺ and equilibrated with Equilibration Buffer (20mM sodium phosphate buffer, 500mM sodium chloride, 5% glycerol, pH 7.8), after loading the protein sample the column was washed with a further 10-20ml of Equilibration Buffer. The column was then washed with Wash Buffer (20mM sodium phosphate buffer, 500mM sodium chloride, 5% glycerol, pH 6.0) until the OD₂₈₀ of the eluate <0.01. Bound protein was eluted using 5ml Elution Buffer (Wash Buffer containing 500mM imidazole, pH 6.0).

The protein was dialysed overnight against Low Salt Buffer (25mM TRJS, 1mM EDTA, 5% glycerol, pH 8.0) and then centrifuged to remove any particulate matter. The protein sample was then loaded onto a Mono-Q column (Pharmacia), which had been pre-equilibrated with Low Salt Buffer. After a 10 column volume wash with Low Salt Buffer, the bound proteins were eluted over 20 column volumes using a gradient between OM sodium chloride to 1M sodium chloride (in 25mM TRIS, 1mM EDTA, 5% glycerol, pH8.0). Peaks on the elution profile were analysed by SDS-PAGE and western blotting.

GH_CP protein was then concentrated (if required) using an Amicon Centriprep Y-10 column.

The purity of the purified GH_CP01 was confirmed by SDS-PAGE, by both coomassie staining and western blot (results not shown). Once the integrity of this sample had been confirmed, GH_CP01 was submitted to the previously established bioassay (Ross *et al*., 1997).

### Example 1

Alternative approach to constructing circular permutations by way of example:
The circular permuted hGH described earlier 
IQTLMGRLED GSPRTGQIFK QTYSKFDTNS HNDDALLKNY GLLYCFRKDM
DKVETFLRIV QCRSVEGSTI PLSRLFDNAS LRAHRLHQLA FDTYQEFEEA
YIPKEQKYSF LQNPQTSLCF SESIPTPSNR EETQQKSNLE LLRISLLLIQ
SWLEPVQFLR SVFANSLVYG ASDSNVYDLL KDLE
 can be encoded by a synthetic gene taking account of optimal codon usage for the expression system. Synthetic genes are readily produced by total gene synthesis. For example the following sequence (producing CP_01) could be constructed for expression in E. coli under control of a suitable promoter (ribosomal binding site underlined: 
1                                                    GAGGACTTAAATTAAATA
   Met Ile Gln Thr Leu Met Gly Arg Leu Glu Asp Gly Ser Pro Arg Thr Gly
17
  19 ATG ATC CAG ACC TTA ATG GGC CGC CTG GAA GAC GGT AGC CCG CGG ACG GGA
   Gln Ile Phe Lys Gln Thr Tyr Ser Lys Phe Asp Thr Asn Ser His Asn Asp
34
  70 CAA ATT TTC AAA CAG ACC TAT AGT AAA TTT GAT ACG AAC AGC CAT AAC GAC
   Asp Ala Leu Leu Lys Asn Tyr Gly Leu Leu Tyr Cys Phe Arg Lys Asp Met
51
 121 GAT GCT CTA CTG AAA AAC TAT GGT CTG CTC TAC TGC TTC CGC AAG GAT ATG
   Asp Lys Val Glu Thr Phe Leu Arg Ile Val Gln Cys Arg Ser Val Glu Gly
68
 172 GAT AAA GTT GAA ACC TTT CTG CGC ATA GTG CAG TGT CGA TCT GTG GAG GGC
   Ser Thr Ile Pro Leu Ser Arg Leu Phe Asp Asn Ala Ser Leu Arg Ala His
85
 223 TCC ACT ATT CCT CTG TCC CGC TTG TTC GAC AAT GCC TCA TTA CGT GCA CAC
   Arg Leu His Gln Leu Ala Phe Asp Thr Tyr Gln Glu Phe Glu Glu Ala Tyr
102
 274 AGA TTG CAT CAG CTT GCC TTT GAT ACG TAC CAG GAG TTT GAA GAA GCG TAT
   Ile Pro Lys Glu Gln Lys Tyr Ser Phe Leu Gln Asn Pro Gln Thr Ser Leu
119
 325 ATT CCG AAG GAG CAA AAA TAC TCT TTT CTG CAA AAT CCG CAG ACC TCG CTG
   Cys Phe Ser Glu Ser Ile Pro Thr Pro Ser Asn Arg Glu Glu Thr Gln Gln
136
 376 TGC TTC AGT GAA AGC ATT CCG ACT CCA TCG AAC CGT GAG GAA ACA CAG CAG
   Lys Ser Asn Leu Glu Leu Leu Arg Ile Ser Leu Leu Leu Ile Gln Ser Trp
153
 427 AAA TCC AAT CTG GAA CTG CTT CGT ATC AGC TTA CTG CTC ATC CAA AGC TGG
   Leu Glu Pro Val Gln Phe Leu Arg Ser Val Phe Ala Asn Ser Leu Val Tyr
170
 478 TTG GAA CCC GTC CAA TTC CTT CGT TCA GTG TTT GCG AAT AGT CTG GTT TAT
   Gly Ala Ser Asp Ser Asn Val Tyr Asp Leu Leu Lys Asp Leu Glu Stop
185
 529 GGG GCA AGT GAC TCT AAC GTC TAT GAT CTG CTG AAA GAT CTC GAA TAA
This could be adapted to carry a suitable purification tag such as a polyhistidine tract incorporated at the C or N-terminus are required to aid in purification.

### Example 2

An alternative permutation CP_02 such as: 
MEIQTLMGRL EDGSPRTGQI FKQTYSKFDT NSHNDDALLK NYGLLYCFRK
DMDKVETFLR IVQCRSVEGS TIPLSRLFDN ASLRAHRLHQ LAFDTYQEFE
EAYIPKEQKY SFLQNPQTSL CFSESIPTPS NREETQQKSN LELLRISLLL
IQSWLEPVQF LRSVFANSLV YGASDSNVYD LLKDL
 in which the break is made one residue earlier than in CP01

CP_02 is encoded by :

1                                                    GAGGACTTAAATTAAATA
    Met Glu Ile Gln Thr Leu Met Gly Arg Leu Glu Asp Gly Ser Pro Arg Thr
17
 19 ATG GAA ATC CAG ACC TTA ATG GGC CGC CTG GAA GAC GGT AGC CCG CGG ACG
    Gly Gln Ile Phe Lys Gln Thr Tyr Ser Lys Phe Asp Thr Asn Ser His Asn
34
 70 GGA CAA ATT TTC AAA CAG ACC TAT AGT AAA TTT GAT ACG AAC AGC CAT AAC
    Asp Asp Ala Leu Leu Lys Asn Tyr Gly Leu Leu Tyr Cys Phe Arg Lys Asp
51
121 GAC GAT GCT CTA CTG AAA AAC TAT GGT CTG CTC TAC TGC TTC CGC AAG GAT
    Met Asp Lys Val Glu Thr Phe Leu Arg Ile Val Gln Cys Arg Ser Val Glu
68
172 ATG GAT AAA GTT GAA ACC TTT CTG CGC ATA GTG CAG TGT CGA TCT GTG GAG
    Gly Ser Thr Ile Pro Leu Ser Arg Leu Phe Asp Asn Ala Ser Leu Arg Ala
85
223 GGC TCC ACT ATT CCT CTG TCC CGC TTG TTC GAC AAT GCC TCA TTA CGT GCA
    His Arg Leu His Gln Leu Ala Phe Asp Thr Tyr Gln Glu Phe Glu Glu Ala
102
274 CAC AGA TTG CAT CAG CTT GCC TTT GAT ACG TAC CAG GAG TTT GAA GAA GCG
    Tyr Ile Pro Lys Glu Gln Lys Tyr Ser Phe Leu Gln Asn Pro Gln Thr Ser
119
325 TAT ATT CCG AAG GAG CAA AAA TAC TCT TTT CTG CAA AAT CCG CAG ACC TCG
    Leu Cys Phe Ser Glu Ser Ile Pro Thr Pro Ser Asn Arg Glu Glu Thr Gln
136
376 CTG TGC TTC AGT GAA AGC ATT CCG ACT CCA TCG AAC CGT GAG GAA ACA CAG
    Gln Lys Ser Asn Leu Glu Leu Leu Arg Ile Ser Leu Leu Leu Ile Gln Ser
153
427 CAG AAA TCC AAT CTG GAA CTG CTT CGT ATC AGC TTA CTG CTC ATC CAA AGC
    Trp Leu Glu Pro Val Gln Phe Leu Arg Ser Val Phe Ala Asn Ser Leu Val
170
478 TGG TTG GAA CCC GTC CAA TTC CTT CGT TCA GTG TTT GCG AAT AGT CTG GTT
    Tyr Gly Ala Ser Asp Ser Asn Val Tyr Asp Leu Leu Lys Asp Leu Stop
185
529 TAT GGG GCA AGT GAC TCT AAC GTC TAT GAT CTG CTG AAA GAT CTC TAA

### Example 3

A further alternative, CP_03 
MQTLMGRLED GSPRTGQIFK QTYSKFDTNS HNDDALLKNY
GLLYCFRKDM DKVETFLRIV QCRSVEGSTI PLSRLFDNAS LRAHRLHQLA
FDTYQEFEEA YIPKEQKYSF LQNPQTSLCF SESIPTPSNR EETQQKSNLE
LLRISLLLIQ SWLEPVQFLR SVFANSLVYG ASDSNVYDLL KDLE
 in which the initial I in CP01 is removed and replaced by the M

Is encoded by :

1                                                    GAGGACTTAAATTAAATA
   Met Gln Thr Leu Met Gly Arg Leu Glu Asp Gly Ser Pro Arg Thr Gly Gln
17
 19 ATG CAG ACC TTA ATG GGC CGC CTG GAA GAC GGT AGC CCG CGG ACG GGA CAA
   Ile Phe Lys Gln Thr Tyr Ser Lys Phe Asp Thr Asn Ser His Asn Asp Asp
34
 70 ATT TTC AAA CAG ACC TAT AGT AAA TTT GAT ACG AAC AGC CAT AAC GAC GAT
   Ala Leu Leu Lys Asn Tyr Gly Leu Leu Tyr Cys Phe Arg Lys Asp Met Asp
51
121 GCT CTA CTG AAA AAC TAT GGT CTG CTC TAC TGC TTC CGC AAG GAT ATG GAT
   Lys Val Glu Thr Phe Leu Arg Ile Val Gln Cys Arg Ser Val Glu Gly Ser
68
172 AAA GTT GAA ACC TTT CTG CGC ATA GTG CAG TGT CGA TCT GTG GAG GGC TCC
   Thr Ile Pro Leu Ser Arg Leu Phe Asp Asn Ala Ser Leu Arg Ala His Arg
85
223 ACT ATT CCT CTG TCC CGC TTG TTC GAC AAT GCC TCA TTA CGT GCA CAC AGA
   Leu His Gln Leu Ala Phe Asp Thr Tyr Gln Glu Phe Glu Glu Ala Tyr Ile
102
274 TTG CAT CAG CTT GCC TTT GAT ACG TAC CAG GAG TTT GAA GAA GCG TAT ATT
   Pro Lys Glu Gln Lys Tyr Ser Phe Leu Gln Asn Pro Gln Thr Ser Leu Cys
119
325 CCG AAG GAG CAA AAA TAC TCT TTT CTG CAA AAT CCG CAG ACC TCG CTG TGC
   Phe Ser Glu Ser Ile Pro Thr Pro Ser Asn Arg Glu Glu Thr Gln Gln Lys
136
376 TTC AGT GAA AGC ATT CCG ACT CCA TCG AAC CGT GAG GAA ACA CAG CAG AAA
   Ser Asn Leu Glu Leu Leu Arg Ile Ser Leu Leu Leu Ile Gln Ser Trp Leu
153
427 TCC AAT CTG GAA CTG CTT CGT ATC AGC TTA CTG CTC ATC CAA AGC TGG TTG
   Glu Pro Val Gln Phe Leu Arg Ser Val Phe Ala Asn Ser Leu Val Tyr Gly
170
478 GAA CCC GTC CAA TTC CTT CGT TCA GTG TTT GCG AAT AGT CTG GTT TAT GGG
   Ala Ser Asp Ser Asn Val Tyr Asp Leu Leu Lys Asp Leu Glu Stop
184
529 GCA AGT GAC TCT AAC GTC TAT GAT CTG CTG AAA GAT CTC GAA TAA

### Example 4

A further variation: Position cysteine residues at C and N termini- so that they may form a disulphide bond, thus making a covalently closed, circular molecule:

CP_04
MCIQTLMGRL EDGSPRTGQI FKQTYSKFDT NSHNDDALLK NYGLLYCFRK
DMDKVETFLR IVQCRSVEGS TIPLSRLFDN ASLRAHRLHQ LAFDTYQEFE
EAYIPKEQKY SFLQNPQTSL CFSESIPTPS NREETQQKSN LELLRISLLL
IQSWLEPVQF LRSVFANSLV YGASDSNVYD LLKDLEC

New cystein residues underlined.
Encoded by:

1                                                    GAGGACTTAAATTAAATA
  Met Cys Ile Gln Thr Leu Met Gly Arg Leu Glu Asp Gly Ser Pro Arg Thr
17
  19 ATG TGT ATC CAG ACC TTA ATG GGC CGC CTG GAA GAC GGT AGC CCG CGG ACG

Gly Gln Ile Phe Lys Gln Thr Tyr Ser Lys Phe Asp Thr Asn Ser His Asn
34
70 GGA CAA ATT TTC AAA CAG ACC TAT AGT AAA TTT GAT ACG AAC AGC CAT AAC
Asp Asp Ala Leu Leu Lys Asn Tyr Gly Leu Leu Tyr Cys Phe Arg Lys Asp
51
121 GAC GAT GCT CTA CTG AAA AAC TAT GGT CTG CTC TAC TGC TTC CGC AAG GAT
Met Asp Lys Val Glu Thr Phe Leu Arg Ile Val Gln Cys Arg Ser Val Glu
68
172 ATG GAT AAA GTT GAA ACC TTT CTG CGC ATA GTG CAG TGT CGA TCT GTG GAG
Gly Ser Thr Ile Pro Leu Ser Arg Leu Phe Asp Asn Ala Ser Leu Arg Ala
85
223 GGC TCC ACT ATT CCT CTG TCC CGC TTG TTC GAC AAT GCC TCA TTA CGT GCA
His Arg Leu His Gln Leu Ala Phe Asp Thr Tyr Gln Glu Phe Glu Glu Ala
102
274 CAC AGA TTG CAT CAG CTT GCC TTT GAT ACG TAC CAG GAG TTT GAA GAA GCG
Tyr Ile Pro Lys Glu Gln Lys Tyr Ser Phe Leu Gln Asn Pro Gln Thr Ser
119
325 TAT ATT CCG AAG GAG CAA AAA TAC TCT TTT CTG CAA AAT CCG CAG ACC TCG
Leu Cys Phe Ser Glu Ser Ile Pro Thr Pro Ser Asn Arg Glu Glu Thr Gln
136
376 CTG TGC TTC AGT GAA AGC ATT CCG ACT CCA TCG AAC CGT GAG GAA ACA CAG
Gln Lys Ser Asn Leu Glu Leu Leu Arg Ile Ser Leu Leu Leu Ile Gln Ser
153
427 CAG AAA TCC AAT CTG GAA CTG CTT CGT ATC AGC TTA CTG CTC ATC CAA AGC
Trp Leu Glu Pro Val Gln Phe Leu Arg Ser Val Phe Ala Asn Ser Leu Val
170
478 TGG TTG GAA CCC GTC CAA TTC CTT CGT TCA GTG TTT GCG AAT AGT CTG GTT
Tyr Gly Ala Ser Asp Ser Asn Val Tyr Asp Leu Leu Lys Asp Leu Glu Cys
187
529 TAT GGG GCA AGT GAC TCT AAC GTC TAT GAT CTG CTG AAA GAT CTC GAA TGT
Stop
187
580 TAA

## Claims

1. A modified human growth hormone polypeptide comprising a modified amino acid sequence which is a modification of the native human growth hormone amino acid sequence, wherein the native amino terminal and carboxyl terminal amino acid residues of native human growth hormone are linked, directly or indirectly, together, wherein the modified human growth hormone is provided with alternative amino terminal and carboxyl terminal amino acid residues and further wherein at least one binding domain of human growth hormone is disrupted.

2. A modified human growth hormone according to claim 1, wherein said native amino terminal and carboxyl terminal amino acid residues are directly linked to each other.

3. A modified human growth hormone according to claim 1, wherein said native amino terminal and carboxyl terminal amino acid residues are indirectly linked by a linking molecule.

4. A modified human growth hormone according to claim 3, wherein said linking molecule is a peptide linker.

5. A modified human growth hormone according to claim 4, wherein said linking peptide is a flexible peptide linker.

6. A modified human growth hormone according to claim 5, wherein said flexible linker is a polypeptide which comprises 5 to 30 amino acid residues.

7. A modified growth hormone according to claim 6, wherein the linker comprises 10 to 20 amino acid residues.

8. A modified human growth hormone according to any of claims 5-7, wherein said linker comprises at least one copy of the peptide: Gly Gly Gly Gly Ser.

9. A modified human growth hormone according to claim 3 or 4, wherein said linker is an inflexible linker.

10. A modified human growth hormone according to claim 9, wherein said linker has, over at least over part of its length, a α-helical region.

11. A modified human growth hormone according to any of claims 1-10, wherein the binding domain of growth hormone comprises a low affinity site 2 receptor binding site.

12. A modified human growth hormone according to claim 11, wherein said low affinity binding domain of growth hormone is between amino acid 116 and amino acid 122 of human growth hormone as represented by the amino acid sequence shown in Figure 1.

13. A modified human growth hormone according to claim 11, wherein the alternative amino terminal and carboxyl terminal amino acid residues are derived from between amino acid 116 and amino acid 122 of human growth hormone as represented by Figure 1.

14. A modified human growth hormone according to claim 11, wherein said alternative amino terminal and carboxyl terminal amino acid residues are derived from between amino acid 118 and amino acid 121 of human growth hormone as represented by Figure 1.

15. A modified human growth hormone according to claim 11, wherein said alternative amino terminal and carboxyl terminal amino acid residues are derived from between amino acid 119 and amino acid 121 of human growth hormone as represented by Figure 1.

16. A modified human growth hormone according to claim 11, wherein said alternative amino terminal and carboxyl terminal amino acid residues are derived from between amino acid 120 and amino acid 121 of human growth hormone as represented by Figure 1.

17. A modified human growth hormone according to claim 11, wherein said alternative amino terminal and carboxyl terminal amino acid residues are derived from between amino acid 118 and amino acid 120 of human growth hormone as represented by Figure 1.

18. A modified human growth hormone according to claim 11, wherein said alternative amino terminal and carboxyl terminal amino acid residues are derived from between amino acid 119 and amino acid 120 of human growth hormone as represented by Figure 1.

19. A modified human growth hormone according to claim 11, wherein said alternative amino terminal and carboxyl terminal amino acid residues are derived from between about amino acid 100 and amino acid 102 of human growth hormone as represented by the amino acid sequence shown in Figure 1.

20. A modified human growth hormone according to claim 11, wherein said alternative amino terminal and carboxyl terminal amino acid residues are derived from between about amino acid 130 and amino acid 132 of human growth hormone as represented by the amino acid sequence shown in Figure 1.

21. An oligomeric cytokine ligand polypeptide comprising at least two modified human growth hormone polypeptides according to any of claims 1-20 wherein said polypeptides are linked, either directly or indirectly, together.

22. An oligomeric cytokine ligand polypeptide according to claim 21 wherein said oligomer consists of two modified human growth hormone polypeptides.

23. An oligomeric cytokine ligand polypeptide according to claim 21 wherein said oligomer comprises at least 3, 4, 5, 6, 7, 8, 9, or at least 10 modified human growth hormone polypeptides.

24. An oligomeric cytokine ligand polypeptide according to claim 21 comprising at least two modified human growth hormone polypeptides linked, either directly or indirectly, to at least one native human growth hormone polypeptide from which said modified human growth hormone polypeptide was derived.

25. An oligomeric cytokine ligand polypeptide according to claim 21 wherein said modified human growth hormone polypeptide is linked to the extracellular ligand binding domain of said ligands cognate receptor.

26. An oligomer cytokine ligand polypeptide according to any of claims 21-25 wherein said linker comprises a cleavage site.

27. An oligomeric cytokine ligand polypeptide according to claim 26 wherein said cleavage site is a proteolytic cleavage site.

28. An oligomeric cytokine ligand polypeptide according to claim 27 wherein said cleavage site is sensitive to a serum protease.

29. An oligomeric cytokine ligand polypeptide according to claim 27 or 28 wherein said cleavage site comprises the amino acid sequence: LVPRGS, or functional variant thereof.

30. An oligomeric cytokine ligand polypeptide according to claim 27 or 28 wherein said cleavage site comprises at least one copy of the amino acid sequence: SGGGG, or functional variant thereof.

31. An oligomeric cytokine ligand polypeptide according to claim 30 wherein said cleavage site comprises the amino acid sequence PGIS.

32. An oligomeric cytokine ligand polypeptide according to claim 30 or 31 wherein said cleavage site comprises the amino acid sequence: LVPRGSPGI.

33. An oligomeric cytokine ligand polypeptide according to claim 32 wherein said cleavage site comprises at least two copies of the amino acid sequence SGGGG that flank said cleavage site.

34. An oligomeric cytokine ligand polyeptide according to claim 33 wherein said cleavage site is sensitive to the serum protease thrombin.

35. A nucleic acid molecule which encodes a modified human growth hormone polypeptide according to any of claims 1-20, or an oligomeric modified human growth hormone polypeptide according to any of claims 21-34.

36. A vector comprising a nucleic acid molecule according to claim 35.

37. A cell transfected or transformed with a nucleic acid molecule or vector according to claim 35 or 36.

38. A cell according to claim 37, wherein said cell is a eukaryotic cell.

39. A cell according to claim 38, wherein said cell is selected from the group consisting of: a mammalian cell; a yeast cell; an insect cell; or a plant cell.

40. A cell according to claim 37, wherein said cell is a prokaryotic cell.

41. A non-human transgenic mammal transfected or transformed with the nucleic acid molecule or vector according to claim 35 or 36.

42. A modified human growth hormone polypeptide according to any of claims 1-20, an oligomeric growth hormone polypeptide according to any of claims 21-34, a nucleic acid molecule or a vector or a cell according to claims 35 or 36 for use as a pharmaceutical.

43. A modified human growth hormone polypeptide according to any of claims 1-20, wherein said polypeptide is fused to the extracellular binding domain of growth hormone receptor.

44. A screening method to generate a modified human growth hormone polypeptide according to claim 1 comprising the steps of:
i) forming a preparation comprising a native human growth hormone polypeptide molecule wherein the native amino terminal and carboxyl terminal amino acids are linked either directly or indirectly together;
ii) generating modified human growth hormone polypeptide molecule wherein said molecule has alternative amino terminal and carboxyl terminal amino acids; and
iii) testing the activity of said modified growth hormone polypeptide in vitro.

## Patentansprüche

1. Modifiziertes humanes Wachstumshormonpolypeptid, umfassend eine modifizierte Aminosäuresequenz, die eine Modifikation der nativen humanen Wachstumshormonaminosäuresequenz ist, wobei die nativen aminoterminalen und carboxyterminalen Aminosäurereste des nativen humanen Wachstumshormons direkt oder indirekt miteinander verknüpft sind, wobei das modifizierte humane Wachstumshormon mit alternativen aminoterminalen und carboxyterminalen Aminosäureresten versehen ist und wobei ferner wenigstens eine Bindungsdomäne des humanen Wachstumshormons zerstört ist.

2. Modifiziertes humanes Wachstumshormon gemäß Anspruch 1, wobei die nativen aminoterminaten und carboxyterminalen Aminosäurereste direkt miteinander verknüpft sind.

3. Modifiziertes humanes Wachstumshormon gemäß Anspruch 1, wobei die nativen aminoterminalen und carboxyterminalen Aminosäurereste indirekt durch ein Linkermolekül verknüpft sind.

4. Modifiziertes humanes Wachstumshormon gemäß Anspruch 3, wobei das Linkermolekül ein Peptidlinker ist.

5. Modifiziertes humanes Wachstumshormon gemäß Anspruch 4, wobei das Linkermolekül ein flexibler Peptidlinker ist.

6. Modifiziertes humanes Wachstumshormon gemäß Anspruch 5, wobei der flexible Linker ein Polypeptid ist, das 5 bis 30 Aminosäurereste umfasst.

7. Modifiziertes Wachstumshormon gemäß Anspruch 6, wobei der Linker 10 bis 20 Aminosäurereste umfasst.

8. Modifiziertes humanes Wachstumshormon gemäß einem der Ansprüche 5 bis 7, wobei der Linker wenigstens eine Kopie des Peptids Gly Gly Gly Gly Ser umfasst.

9. Modifiziertes humanes Wachstumshormon gemäß Anspruch 3 oder 4, wobei der Linker ein unflexibler Linker ist.

10. Modifiziertes humanes Wachstumshormon gemäß Anspruch 9, wobei der Linker über wenigstens einen Teil seiner Länge eine α-helikale Region hat.

11. Modifiziertes humanes Wachstumshormon gemäß einem der Ansprüche 1 bis 10, wobei die Bindungsdomäne des Wachstumshormons eine niederaffine Bindungsstelle 2 als Rezeptorbindungsstelle umfasst.

12. Modifiziertes humanes Wachstumshormon gemäß Anspruch 11, wobei die niederaffine Bindungsdomäne des Wachstumshormons sich zwischen Aminosäure 116 und Aminosäure 122 des humanen Wachstumshormons befindet, wie es durch die in Figur 1 gezeigte Aminosäuresequenz dargestellt wird.

13. Modifiziertes humanes Wachstumshormon gemäß Anspruch 11, wobei die alternativen aminoterminalen und carboxyterminalen Aminosäurereste von Aminosäure 116 bis Aminosäure 122 des humanen Wachstumshormons abstammen, wie es in Figur 1 dargestellt wird.

14. Modifiziertes humanes Wachstumshormon gemäß Anspruch 11, wobei die alternativen aminoterminalen und carboxyterminalen Aminosäurereste von Aminosäure 118 bis Aminosäure 121 des humanen Wachstumshormons abstammen, wie es in Figur 1 dargestellt wird.

15. Modifiziertes humanes Wachstumshormon gemäß Anspruch 11, wobei die alternativen aminoterminalen und carboxyterminalen Aminosäurereste von Aminosäure 119 bis Aminosäure 121 des humanen Wachstumshormons abstammen, wie es in Figur 1 dargestellt wird.

16. Modifiziertes humanes Wachstumshormon gemäß Anspruch 11, wobei die alternativen aminoterminalen und carboxyterminalen Aminosäurereste von Aminosäure 120 bis Aminosäure 121 des humanen Wachstumshormons abstammen, wie es in Figur 1 dargestellt wird.

17. Modifiziertes humanes Wachstumshormon gemäß Anspruch 11, wobei die alternativen aminoterminalen und carboxyterminalen Aminosäurereste von Aminosäure 118 bis Aminosäure 120 des humanen Wachstumshormons abstammen, wie es in Figur 1 dargestellt wird.

18. Modifiziertes humanes Wachstumshormon gemäß Anspruch 11, wobei die alternativen aminoterminalen und carboxyterminalen Aminosäurereste von Aminosäure 119 bis Aminosäure 120 des humanen Wachstumshormons abstammen, wie es in Figur 1 dargestellt wird.

19. Modifiziertes humanes Wachstumshormon gemäß Anspruch 11, wobei die alternativen aminoterminalen und carboxyterminalen Aminosäurereste von ungefähr Aminosäure 100 bis Aminosäure 102 des humanen Wachstumshormons abstammen, wie es durch die in Figur 1 gezeigte Aminosäuresequenz dargestellt wird.

20. Modifiziertes humanes Wachstumshormon gemäß Anspruch 11, wobei die alternativen aminoterminalen und carboxyterminalen Aminosäurereste von ungefähr Aminosäure 130 bis Aminosäure 132 des humanen Wachstumshormons abstammen, wie es durch die in Figur 1 gezeigte Aminosäuresequenz dargestellt wird.

21. Oligomeres Zytokinligandenpolypeptid, umfassend wenigstens zwei modifizierte humane Wachstumshormon polypeptide gemäß einem der Ansprüche 1 bis 20, wobei die Polypeptide direkt oder indirekt miteinander Verknüpft sind.

22. Oligomeres Zytokinligandenpolypeptid gemäß Anspruch 21, wobei das Oligomer aus zwei modifizierten humanen Wachstumshormonpolypeptiden besteht.

23. Oligomeres Zytokinligandenpolypeptid gemäß Anspruch 21, wobei das Oligomer wenigstens 3, 4, 5, 6, 7, 8, 9 oder wenigstens 10 modifizierte humane Wachstumshormonpolypeptide umfasst.

24. Oligomeres Zytokinligandenpolypeptid gemäß Anspruch , 21, umfassend wenigstens zwei modifizierte humane Wachstumshormonpolypeptide, die direkt oder indirekt mit wenigstens einem nativen humanen Wachstumshormonpolypeptid verknüpft sind, von dem sich das modifizierte humane Wachstumshormonpolypeptid ableitet.

25. Oligomeres Zytokinligandenpolypeptid gemäß Anspruch 21, wobei das modifizierte humane Wachstumshormonpolypeptid mit der extrazellulären Ligandenbindungsdomäne des Ligandenrezeptors verknüpft sind.

26. Oligomeres Zytokinligandenpolypeptid gemäß einem der Ansprüche 21 bis 25, wobei der Linker eine Schnittstelle umfasst.

27. Oligomeres Zytokinligandenpolypeptid gemäß Anspruch 26, wobei die Schnittstelle eine proteolytische Schnittstelle ist.

28. Oligomeres Zytokinligandenpolypeptid gemäß Anspruch 27, wobei die Schnittstelle spezifisch für Serumproteasen ist.

29. Oligomeres Zytokinligandenpolypeptid gemäß Anspruch 27 oder 28, wobei die Schnittstelle die Aminosäuresequenz LVPRGS oder eine funktionelle Variante davon umfasst.

30. Oligomeres Zytokinligandenpolypeptid gemäß Anspruch 27 oder 28, wobei die Schnittstelle wenigstens eine Kopie der Aminosäuresequenz SGGGG oder eine funktionellen Variante davon umfasst.

31. Oligomeres Zylokinligandenpolypeptid gemäß Anspruch 30, wobei die Schnittstelle die Aminosäuresequenz PGIS umfasst.

32. Oligomeres Zytokinligandenpolypeptid gemäß Anspruch 30 oder 31, wobei die Schnittstelle die Aminosäuresequenz LVPRGSPC51 umfasst.

33. Oligomeres Zytokinligandenpolypeptid gemäß Anspruch 32, wobei die Schnittstelle wenigstens zwei Kopien der Aminosäuresequenz SGGGG umfasst, die die Schnittstelle flankieren,

34. Oligomeres Zytokinligandenpolypeptid gemäß Anspruch 33, wobei die Schnittstelle spezifisch für die Serumprotease Thrombin ist.

35. Nukleinsäuremolekül, das ein modifiziertes humanes Wachstumshormonpolypeptid gemäß einem der Ansprüche 1 bis 20 oder ein oligomeres modifiziertes humanes Wachstumshormonpolypeptid gemäß einem der Ansprüche 21 bis 34 codiert.

36. Vektor, umfassend ein Nukleinsäutemolekül gemäß Anspruch 35.

37. Zelle, transfiziert oder transformiert mit einem Nukleinsäuremolekül oder einem Vektor gemäß Anspruch 35 oder 36.

38. Zelle gemäß Anspruch 37, wobei die Zelle eine eukaryontische Zelle ist.

39. Zelle gemäß Anspruch 38, wobei die Zelle ausgewählt ist aus der Gruppe bestehend aus einer Säugerzelle, einer Hefezelle, einer Insektenzelle oder einer Pflanzenzelle.

40. Zelle gemäß Anspruch 37, wobei die Zelle eine prokaryontische Zelle ist.

41. Nichthumaner transgener Säuger, transfiziert oder transformiert mit dem Nukleinsäuremolekül oder dem Vektor gemäß Anspruch 35 oder 36.

42. Modifiziertes humanes Wachstumshormonpolypeptid gemäß einem der Ansprüche 1 bis 20, oligomeres Wachstumshormonpolypeptid gemäß einem der Ansprüche 21 bis 34, Nukleinsäuremolekül oder Vektor oder Zelle gemäß einem der Ansprüche 35 oder 36 zur Verwendung als Pharmazeutikum.

43. Modifiziertes humanes Wachstumshormönpolypeptid gemäß einem der Ansprüche 1 bis 20, wobei das Polypeptid mit der extrazellulären Bindungsdomäne des Wachstumshormonrezeptors fusioniert ist.

44. Screeningverfahren zum Erzeugen eines modifizierten humanen Wachstumshormonpolypeptids gemäß Anspruch 1, umfassend die Schritte:
i) Herstellen eines Präparats, umfassend ein natives humanes Wachstumshormonpolypeptidmolekül, wobei die nativen aminoterminalen und carboxyterminalen Aminosäuren direkt oder indirekt miteinander verknüpft sind;
ii) Erzeugen eines modifizierten humanen Wachsturnshormenpolypeptidmoleküls, wobei das Molekül alternative aminoterminale und carboxyterminale Aminosäuren hat; und
iii) Testen der Aktivität des modifizierten humanen Wachstumshormonpolypeptids *in vitro.*

## Revendications

1. Polypeptide d'hormone de croissance humaine modifiée comprenant une séquence d'acides aminés modifiée qui est une modification de la séquence d'acides aminés d'hormone de croissance humaine native, dans lequel les résidus d'acides aminés amino terminal et carboxyle terminal natifs de l'hormone de croissance humaine native sont liés, ensemble, directement ou indirectement, où l'hormone de croissance humaine modifiée est pourvue de résidus d'acides aminés amino terminal et carboxyle terminal alternatifs et en outre où au moins un domaine de liaison de l'hormone de croissance humaine est rompu.

2. Hormone de croissance humaine modifiée selon la revendication 1, dans laquelle lesdits résidus d'acides aminés amino terminal et carboxyle terminal natifs sont directement liés l'un à l'autre.

3. Hormone de croissance humaine modifiée selon la revendication 1, dans laquelle lesdits résidus d'acides aminés amino terminal et carboxyle terminal natifs sont indirectement liés par une molécule de liaison.

4. Hormone de croissance humaine modifiée selon la revendication 3, dans laquelle ladite molécule de liaison est peptide.

5. Hormone de croissance humaine modifiée selon la revendication 4, dans laquelle ledit peptide de liaison est un est peptide flexible.

6. Hormone de croissance humaine modifiée selon la revendication 5, dans laquelle ledit peptide flexible est un polypeptide qui comprend 5 à 30 résidus d'acides aminés.

7. Hormone de croissance humaine modifiée selon la revendication 6, dans laquelle le peptide comprend 10 à 20 résidus d'acides aminés.

8. Hormone de croissance humaine modifiée selon l'une quelconque des revendications 5 à 7, dans laquelle ledit peptide comprend au moins une copie du peptide : Gly Gly Gly Gly Ser.

9. Hormone de croissance humaine modifiée selon la revendication 3 ou 4, dans laquelle ledit peptide est un peptide inflexible.

10. Hormone de croissance humaine modifiée selon la revendication 9, dans laquelle ledit peptide a, sur au moins une partie de sa longueur, une région α-hélicoïdale.

11. Hormone de croissance humaine modifiée selon l'une quelconque des revendications 1 à 10, dans laquelle le domaine de liaison de l'hormone de croissance comprend un site 2 de liaison au récepteur de faible affinité.

12. Hormone de croissance humaine modifiée selon la revendication 11, dans laquelle ledit domaine de liaison de faible affinité de l'hormone de croissance est entre l'acide aminé 116 et l'acide aminé 122 de l'hormone de croissance humaine telle que représentée par la séquence d'acides aminés montrée sur la figure 1.

13. Hormone de croissance humaine modifiée selon la revendication 11, dans laquelle les résidus d'acides aminés amino terminal et carboxyle terminal alternatifs sont dérivés d'entre l'acide aminé 116 et l'acide aminé 122 de l'hormone de croissance humaine telle que représentée par la figure 1.

14. Hormone de croissance humaine modifiée selon la revendication 11, dans laquelle lesdits résidus d'acides aminés amino terminal et carboxyle terminal alternatifs sont dérivés d'entre l'acide aminé 118 et l'acide aminé 121 de l'hormone de croissance humaine telle que représentée par la figure 1.

15. Hormone de croissance humaine modifiée selon la revendication 11, dans laquelle lesdits résidus d'acides aminés amino terminal et carboxyle terminal alternatifs sont dérivés d'entre l'acide aminé 119 et l'acide aminé 121 de l'hormone de croissance humaine telle que représentée par la figure 1.

16. Hormone de croissance humaine modifiée selon la revendication 11, dans laquelle lesdits résidus d'acides aminés amino terminal et carboxyle terminal alternatifs sont dérivés d'entre l'acide aminé 120 et l'acide aminé 121 de l'hormone de croissance humaine telle que représentée par la figure 1.

17. Hormone de croissance humaine modifiée selon la revendication 11, dans laquelle lesdits résidus d'acides aminés amino terminal et carboxyle terminal alternatifs sont dérivés d'entre l'acide aminé 118 et l'acide aminé 120 de l'hormone de croissance humaine telle que représentée par la figure 1.

18. Hormone de croissance humaine modifiée selon la revendication 11, dans laquelle lesdits résidus d'acides aminés amino terminal et carboxyle terminal alternatifs sont dérivés d'entre l'acide aminé 119 et l'acide aminé 120 de l'hormone de croissance humaine telle que représentée par la figure 1.

19. Hormone de croissance humaine modifiée selon la revendication 11, dans laquelle lesdits résidus d'acides aminés amino terminal et carboxyle terminal alternatifs sont dérivés d'entre environ l'acide aminé 100 et l'acide aminé 102 de l'hormone de croissance humaine telle que représentée par la figure 1.

20. Hormone de croissance humaine modifiée selon la revendication 11, dans laquelle lesdits résidus d'acides aminés amino terminal et carboxyle terminal alternatifs sont dérivés d'entre environ l'acide aminé 130 et l'acide aminé 132 de l'hormone de croissance humaine telle que représentée par la figure 1.

21. Polypeptide oligomérique de ligand de cytokine comprenant au moins deux polypeptides d'hormone de croissance humaine modifiée selon l'une quelconque des revendications 1 à 20, dans lequel lesdits polypeptides sont liés, ensemble, soit directement, soit indirectement.

22. Polypeptide oligomérique de ligand de cytokine selon la revendication 21, dans lequel ledit oligomère est constitué de deux polypeptides d'hormone de croissance humaine modifiée.

23. Polypeptide oligomérique de ligand de cytokine oligomère selon la revendication 21, dans lequel ledit oligomère comprend au moins 3, 4, 5, 6, 7, 8, 9 ou au moins 10 polypeptides d'hormone de croissance humaine modifiée.

24. Polypeptide oligomérique de ligand de cytokine selon la revendication 21, comprenant au moins deux polypeptides d'hormone de croissance humaine modifiée liés, soit directement, soit indirectement, à au moins un polypeptide d'hormone de croissance humaine native à partir duquel ledit polypeptide d'hormone de croissance humaine modifiée a été dérivé.

25. Polypeptide oligomérique de ligand de cytokine selon la revendication 21, dans lequel ledit polypeptide d'hormone de croissance humaine modifiée est lié au domaine de liaison de ligand extracellulaire du récepteur parent desdits ligands.

26. Polypeptide oligomérique de ligand de cytokine selon l'une quelconque des revendications 21 à 25, dans lequel ledit lieur comprend un site de clivage.

27. Polypeptide oligomérique de ligand de cytokine selon la revendication 26. dans lequel ledit site de clivage est un site de clivage protéolytique.

28. Polypeptide oligomérique de ligand de cytokine selon la revendication 27. dans lequel ledit site de clivage est sensible à une protéase sérique.

29. Polypeptide oligomérique de ligand de cytokine selon la revendication 27 ou 28, dans lequel ledit site de clivage comprend la séquence d'acides aminés : LVPRGS, ou une variante fonctionnelle de celle-ci.

30. Polypeptide oligomérique de ligand de cytokine selon la revendication 27 ou 28, dans lequel ledit site de clivage comprend au moins une copie de la séquence d'acides aminés : SGGGG, ou une variante fonctionnelle de celle-ci.

31. Polypeptide de ligand de cytokine oligomère selon la revendication 30, dans lequel ledit site de clivage comprend la séquence d'acides aminés PGIS.

32. Polypeptide de ligand de cytokine oligomère selon la revendication 30 ou 31, dans lequel ledit site de clivage comprend la séquence d'acides aminés : LVPRGSPGI.

33. Polypeptide oligomérique de ligand de cytokine selon la revendication 32, dans lequel ledit site de clivage comprend au moins deux copies de la séquence d'acides aminés : SGGGG qui flanque ledit site de clivage,

34. Polypeptide oligomérique de ligand de cytokine oligomère selon la revendication 33, dans lequel ledit site de clivage est sensible au sérum protéase thrombine.

35. Molécule d'acide nucléique qui code un polypeptide d'hormone de croissance humaine modifiée selon l'une quelconque des revendications 1 à 20, ou un polypeptide oligomérique d'hormone de croissance humaine modifiée selon l'une quelconque des revendications 21 à 34.

36. Vecteur comprenant une molécule d'acide nucléique selon la revendication 35.

37. Cellule transfectée ou transformée avec une molécule d'acide nucléique ou un vecteur selon la revendication 35 ou 36.

38. Cellule selon la revendication 37, dans laquelle ladite cellule est une cellule eucaryote.

39. Cellule selon la revendication 38, dans laquelle ladite cellule est choisie dans le groupe consistant en : une cellule de mammifère ; une cellule de levure ; une cellule d'insecte ; ou une cellule végétale.

40. Cellule selon la revendication 37, dans laquelle ladite cellule est une cellule procaryote.

41. Mammifère transgénique non humain transfecté ou transformé avec la molécule d'acide nucléique ou le vecteur selon la revendication 35 ou 36.

42. Polypeptide d'hormone de croissance humaine modifiée selon l'une quelconque des revendications 1 à 20, polypeptide oligomérique d'hormone de croissance selon l'une quelconque des revendications 21 à 34, molécule d'acides nucléiques ou vecteur ou cellule selon les revendications 35 à 36, pour son utilisation comme produit pharmaceutique,

43. Polypeptide d'hormone de croissance humaine modifiée selon l'une quelconque des revendications 1 à 20, dans lequel ledit polypeptide est fusionné au domaine de liaison extracellulaire du récepteur d'hormone de croissance.

44. Procédé de criblage pour générer un polypeptide d'hormone de croissance humaine modifiée selon la revendication 1, comprenant les étapes suivantes :
i) former une préparation comprenant une molécule de polypeptide d'hormone de croissance humaine native dans laquelle les acides aminés amino terminal et carboxyle terminal natifs sont liés soit directement, soit indirectement ensemble ;
ii) générer la molécule de polypeptide d'hormone de croissance humaine modifiée où ladite molécule comporte des acides aminés amino terminal et carboxyle terminal alternatifs ; et
iii) évaluer l'activité dudit polypeptide d'hormone de croissance modifiée *in vitro*.
